# EUROPEAN PATENT APPLICATION

(11) **EP 4 692 296 A1**
(43) Date of publication of application: **11.02.2026**
(21) Application number: 23930941.2
(22) Date of filing: 21.12.2023
(51) Int. Cl.: C12M 1/00, C12M 1/34, C12N 1/00

(54) **CELL CULTURE DEVICE, CULTURE CONDITIONS DETERMINATION DEVICE, AND CULTURE CONDITIONS DETERMINATION METHOD**

(30) Priority: 29.03.2023 JP 2023053523
(71) Applicant: Hitachi Plant Services Co., Ltd., Tokyo 170-6034 (JP)
(72) Inventor: OKA Kenichiro, Tokyo 100-8280 (JP); SHIBUYA Keisuke, Tokyo 100-8280 (JP)
(74) Representative: MERH-IP Matias Erny Reichl Hoffmann Patentanwälte PartG mbB
(86) International application number: PCT/JP2023/045935
(87) International publication number: WO 2024/202322

(57) **Abstract**

A search for an optimal culture condition is to obtain an optimal culture condition based on a combination of explanatory variables in which an objective variable becomes maximum or minimum, that is, the explanatory variables. A next experiment condition is determined using an explanatory variable that is a measurement value requiring a long time for measurement and a substitute value that is a measurement value capable of performing short-time measurement among the explanatory variables, thereby obtaining the optimal culture condition based on the substitute value in a short time. At the same time, an explanatory variable that is a measurement value requiring a long time for measurement is measured, a correlation database between the explanatory variable and the substitute value is constructed, and the optimal culture condition based on the substitute value is converted to obtain an optimal culture condition based on the explanatory variable.

## Description

### Technical Field

The present invention relates to a cell culture device, a culture condition determination device, and a culture condition determination method.

### Background Art

PTL 1 discloses a technique related to the invention. PTL 1 describes that "a target value of an attitude angle of the predetermined component is calculated based on a fuel consumption rate or a substitute value thereof and a statistical model generated by the statistical model generation unit". PTL 1 describes that "the substitute value of the fuel consumption rate includes at least one of a fuel consumption amount, an engine speed, a traveling speed of the ship, and a throttle opening degree".

### Citation List

### Patent Literature

PTL 1: JP2004-299529A

### Summary of Invention

### Technical Problem

A function form, in which each of a plurality of culture conditions (a plurality of parameters) obtained as measurement data is an explanatory variable and a specific measurement value related to cell culture is an objective variable, is obtained, and an explanatory variable (an optimal culture condition) in which the objective variable is maximized or minimized is determined.

For example, in production of an antibody drug, cells are grown using a medium placed in a culture tank, and antibody proteins produced in the cells are purified.

A growth rate of cells and a yield of the produced antibody proteins are determined by the culture conditions in cell culture. Examples of the culture conditions to be considered include a temperature, a mixing performance, a gas exchange performance, a dissolved oxygen concentration, a dissolved carbon dioxide concentration, a pH, and a shear stress obtained as the measurement data.

When the optimal culture condition is determined, it is necessary to search for a combination of culture conditions (a plurality of parameters) that maximizes or minimizes the objective variable in culture.

However, a trial-and-error repeated experiment is required to search for the optimal culture condition that maximizes or minimizes the objective variable, and there is a problem that the experiment cost and time are required.

In the trial-and-error repeated experiment, it is necessary to measure a culture state during the experiment and a culture result after the end of the experiment, and determine the following experiment conditions based on the measurement results. When an item to be measured according to the next experiment condition determination requires a long time for measurement, there is a problem that a total time of the repeated experiment is long.

In PTL 1, control is performed using the substitute value, but it is not possible to obtain a function notation expressing a relationship between the explanatory variable that is a source of the substitute value and the objective variable.

The invention is made to solve the above problems. That is, an object of the invention is to provide a cell culture device, a culture condition determination device, and a culture condition determination method capable of quickly determining an optimal culture condition even when it takes time to measure a parameter for obtaining the optimal culture condition of cell culture.

### Solution to Problem

In order to solve the above problems, a cell culture device of the invention includes a culture tank and a culture solution containing cells inside the culture tank. The cell culture device includes: a first measurement device configured to measure a first parameter that is a culture condition and a second parameter related to a result of a culture experiment; a second measurement device configured to measure a substitute parameter that is at least one of a first substitute value of the first parameter and a second substitute value of the second parameter; and an arithmetic device configured to search for an optimal culture condition. The arithmetic device: acquires the first parameter and the second parameter from the first measurement device and acquires the substitute parameter from the second measurement device; based on the first parameter, the second parameter, and the substitute parameter, obtains a function form expressed using the substitute parameter, the function form using the first parameter as an explanatory variable and the second parameter as an objective variable; calculates a parameter correlation between the substitute parameter and at least one of the first parameter and the second parameter substituted by the substitute parameter; calculates an optimal explanatory variable in which the objective variable of the function form expressed using the substitute parameter becomes maximum or minimum; calculates the calculated optimal explanatory variable as an optimal culture condition when the function form expressed using the substitute parameter is not a function form expressed using the first substitute value; and converts the calculated optimal explanatory variable into an optimal first parameter based on the parameter correlation and calculates the optimal first parameter as the optimal culture condition when the function form expressed using the substitute parameter is the function form expressed using at least the first substitute value.

A culture condition determination device of the invention includes an arithmetic device configured to acquire, from a first measurement device that measures a first parameter that is a culture condition and a second parameter related to a result of a culture experiment and from a second measurement device that measures a substitute parameter that is at least one of a first substitute value of the first parameter and a second substitute value of the second parameter, the first parameter, the second parameter, and the substitute parameter, and search for an optimal culture condition. The arithmetic device: obtains, based on the first parameter, the second parameter, and the substitute parameter, a function form expressed using the substitute parameter, the function form using the first parameter as an explanatory variable and the second parameter as an objective variable; calculates a parameter correlation between the substitute parameter and at least one of the first parameter and the second parameter substituted by the substitute parameter; calculates an optimal explanatory variable in which the objective variable of the function form expressed using the substitute parameter becomes maximum or minimum; calculates the calculated optimal explanatory variable as an optimal culture condition when the function form expressed using the substitute parameter is not a function form expressed using the first substitute value; and converts the optimal explanatory variable into an optimal first parameter based on the parameter correlation and calculates the optimal first parameter as the optimal culture condition when the function form expressed using the substitute parameter is the function form expressed using at least the first substitute value.

A culture condition determination method of the invention is a culture condition determination method of cell culture by a culture tank and a culture solution containing cells inside the culture tank. By using a first measurement device configured to measure a first parameter that is a culture condition and a second parameter related to a result of a culture experiment, a second measurement device configured to measure a substitute parameter that is at least one of a first substitute value of the first parameter and a second substitute value of the second parameter, and an arithmetic device configured to search for an optimal culture condition, the culture condition determination method includes by the arithmetic device: acquiring the first parameter and the second parameter from the first measurement device and acquiring the substitute parameter from the second measurement device; based on the first parameter, the second parameter, and the substitute parameter, obtaining a function form expressed using the substitute parameter, the function form using the first parameter as an explanatory variable and the second parameter as an objective variable; calculating a parameter correlation between the substitute parameter and at least one of the first parameter and the second parameter substituted by the substitute parameter; calculating an optimal explanatory variable in which the objective variable of the function form expressed using the substitute parameter becomes maximum or minimum; calculating the calculated optimal explanatory variable as an optimal culture condition when the function form expressed using the substitute parameter is not a function form expressed using the first substitute value; and converting the calculated optimal explanatory variable into an optimal first parameter based on the parameter correlation and calculating the optimal first parameter as the optimal culture condition when the function form expressed using the substitute parameter is the function form expressed using at least the first substitute value.

### Advantageous Effects of Invention

According to the invention, even when it takes time to measure a parameter for obtaining an optimal culture condition of cell culture, the optimal culture condition can be quickly determined.

### Brief Description of Drawings

[FIG. 1] FIG. 1 is a diagram showing an optimal culture condition search procedure in the related art.
[FIG. 2] FIG. 2 is a diagram showing an optimal culture condition procedure according to an embodiment of the invention.
[FIG. 3] FIG. 3 is a diagram showing a formula.
[FIG. 4] FIG. 4 is a diagram showing an example of a configuration of a cell culture device according to the embodiment of the invention.
[FIG. 5] FIG. 5 is a diagram showing a hardware structure example of an arithmetic device.
[FIG. 6] FIG. 6 is a diagram showing an optimal culture condition procedure of a first modification.
[FIG. 7] FIG. 7 is a diagram showing a formula.
[FIG. 8] FIG. 8 is a diagram showing an optimal culture condition procedure of a second modification.
[FIG. 9] FIG. 9 is a diagram showing a formula.

### Description of Embodiments

Hereinafter, embodiments of the invention will be described with reference to the drawings. In the drawings of the embodiments, the same or corresponding parts may be denoted by the same reference numerals.

A culture device of biological cells (also referred to as a "cell culture device") according to the embodiment of the invention can be applied when culturing cells that produce a substance serving as a main raw material of a drug or the like.

In the embodiment of the invention, the substance to be produced is not particularly limited, and examples thereof include proteins such as antibodies and enzymes, and physiologically active substances such as low molecular compounds and high molecular compounds. The cells to be cultured are not particularly limited, and examples thereof include animal cells, plant cells, insect cells, bacteria, yeast, fungi, and algae. In particular, the culture device of the biological cells preferably targets animal cells that produce proteins such as antibodies and enzymes.

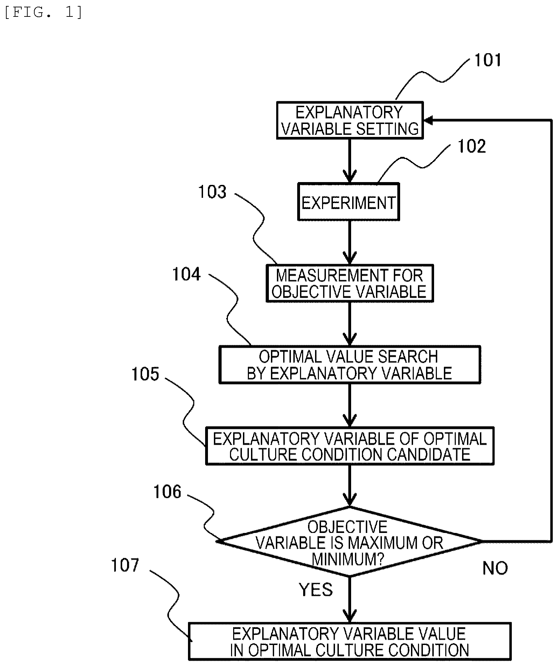

First, in order to facilitate understanding of the invention, an explanatory variable value determination method in an optimal culture condition based on a method in the related art will be described with reference to FIG. 1. In FIG. 1, an objective variable is a target value to be maximized or minimized in cell culture, and corresponds to a cell growth rate, a yield of produced antibody proteins, or the like. An explanatory variable refers to a culture condition in cell culture, and examples thereof include a temperature, a mixing performance, a gas exchange performance, a dissolved oxygen concentration, a dissolved carbon dioxide concentration, a pH, and a shear stress. In a method in the related art, each of a plurality of culture conditions is used as an explanatory variable, a function form using a specific measurement value (such as a cell growth rate or a yield of a produced antibody proteins) as the objective variable is obtained, and an optimal culture condition in which the objective variable is maximum or minimum is determined.

As shown in FIG. 1, in the method in the related art, first, an explanatory variable setting 101 as a culture condition is performed, and an experiment 102 is performed. The experiment 102 referred to here is to perform culture in the setting of the explanatory variable setting 101. After the end of the experiment 102, a measurement 103 for the objective variable is performed.

Based on one or more sets of the explanatory variable setting 101 and a result of the measurement 103 for the objective variable, a mathematical method is used to obtain a function notation of the objective variable using the explanatory variable as a dependent variable. As the mathematical method here, a plurality of methods such as linear regression, kernel regression, and Gaussian process regression can be applied.

By using the function notation of the objective variable acquired above and using the mathematical method, an optimal value search 104 by an explanatory variable is performed to determine an explanatory variable 105 of an optimal culture condition candidate. As the mathematical method here, a Monte Carlo simulation, a genetic algorithm, an annealing method, a Bayesian optimization method, or the like can be applied.

In a determination 106 for the objective variable, a procedure from the explanatory variable setting 101 to the explanatory variable 105 of the optimal culture condition candidate is repeated until the objective variable becomes maximum or minimum. An explanatory variable value in which the objective variable becomes maximum or minimum is an explanatory variable value 107 in the optimal culture condition.

In the method in the related art, an optimal culture condition is determined as described above. However, in the method in the related art, when a culture condition to be obtained includes a parameter (physical quantity) that takes a long time for measurement, there is a problem that a total time of the repeated experiments required until the optimal culture condition is obtained is long.

Therefore, in the cell culture device according to the embodiment of the invention, an optimal culture condition is determined by the explanatory variable value determination method in the optimal culture condition as described below.

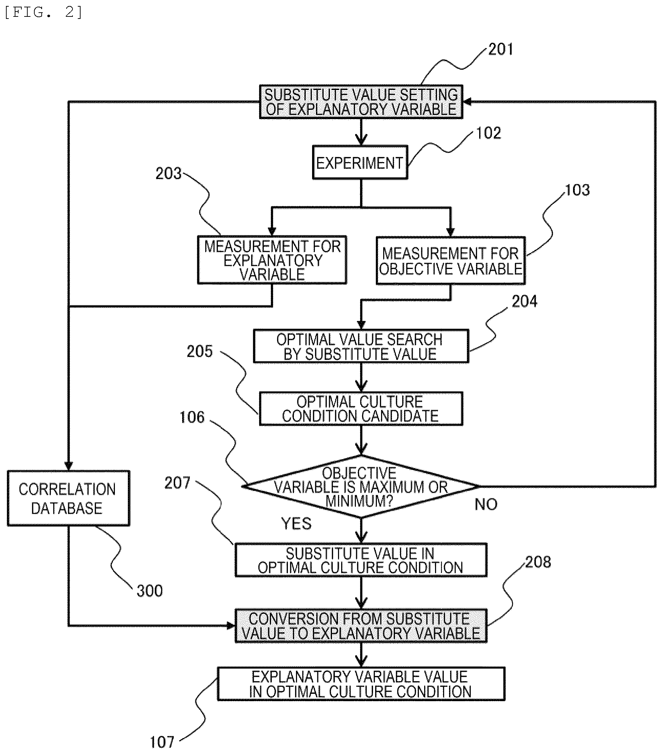

With reference to FIG. 2, the explanatory variable value determination method in the optimal culture condition performed by the cell culture device according to the embodiment of the invention will be described. In FIG. 2, the objective variable is a target value to be maximized or minimized in cell culture, and corresponds to a cell growth rate, a yield of produced antibody proteins, or the like. The explanatory variable refers to a culture condition in cell culture, and examples thereof include a temperature distribution, a mixing performance, a gas exchange performance, a dissolved oxygen concentration distribution, a dissolved carbon dioxide concentration distribution, a pH distribution, a shear stress distribution, and a glucose concentration in a culture tank. A substitute value of the explanatory variable is a temperature sensor value, a dissolved oxygen concentration sensor value, a dissolved carbon dioxide concentration sensor value, a pH sensor value, a stirring blade rotation speed, and a glucose concentration in an added medium.

In the invention, first, instead of the explanatory variable that is the culture condition, a substitute value setting 201 of the explanatory variable is performed, and an experiment 102 is performed. The experiment 102 referred to here is to perform culture in the setting of the substitute value setting 201 of the explanatory variable. After the end of the experiment 102, the measurement 103 for the objective variable is performed.

A measurement 203 for the explanatory variable is performed before, after, or simultaneously with the measurement 103 for the objective variable. The measurement here includes not only biochemical measurement but also calculation using a result of numerical fluid analysis.

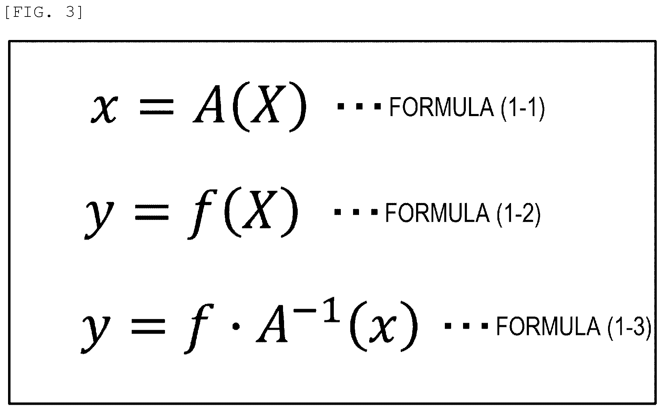

A correlation between the substitute value setting 201 of the explanatory variable and the result of the measurement 203 for the explanatory variable is calculated to create a correlation database 300. Methods such as multiple linear regression measurement, nonlinear kernel regression measurement, and support vector regression can be applied to the calculation of the correlation. When a vector in which a plurality of substitute values are arranged is X and a vector in which a plurality of explanatory variables are arranged is x, a relationship therebetween can be expressed by Formula (1-1) in FIG. 3.

Based on of one or more sets of the substitute value setting 201 of the explanatory variable and the result of the measurement 103 for the objective variable, an optimal value search 204 by the substitute value is performed by using the mathematical method to obtain a function notation of the objective variable using the substitute value as a dependent variable. As the mathematical method here, a plurality of methods such as linear regression, kernel regression, and Gaussian process regression can be applied. When the objective variable is y and the vector in which the plurality of substitute values are arranged is X, a relationship therebetween can be expressed by Formula (1-2) in FIG. 3.

By using the function notation (Formula (1-2) in FIG. 3) acquired as described above, a substitute value for the next experiment, which is an optimal culture condition candidate 205, is determined using the mathematical method. As the mathematical method here, a Monte Carlo simulation, a genetic algorithm, an annealing method, a Bayesian optimization method, or the like can be applied.

In the determination 106 for the objective variable, a procedure from the substitute value setting 201 of the explanatory variable to the determination of the optimal culture condition candidate 205 is repeated until the objective variable becomes maximum or minimum. The explanatory variable value in which the objective variable becomes maximum or minimum is a substitute value 207 in the optimal culture condition.

The substitute value 207 in the optimal culture condition is converted using the correlation database 300 to obtain the explanatory variable value 107 in the optimal culture condition. At the same time, a function notation of the objective variable expressed by Formula (1-3) in FIG. 3 using the explanatory variable as a dependent variable is obtained by Formula (1-1) in FIG. 3 and Formula (1-2) in FIG. 3.

Here, in order to deepen the understanding of the invention, an inventive step of an optimal culture condition determination method using the substitute value in the invention will be described by taking the glucose concentration in the culture tank, which is an explanatory variable, and the glucose concentration in the added medium, which is a substitute value, as an example. Glucose is a nutrient source of cells, and cells are active by consuming glucose. Therefore, an index that affects the activity of cells is the glucose concentration in the culture tank. However, the glucose concentration in the culture tank cannot be measured in real time, and it is necessary to appropriately extract a part of a culture solution and measure the glucose concentration by a measurement device outside the culture tank. On the other hand, the glucose concentration in the added medium, which is a substitute value, can be controlled to any value by adding an appropriate amount of a high-concentration glucose aqueous solution to a standard added medium. The function notation of the objective variable using the substitute value as the dependent variable is as in Formula (1-2) in FIG. 3. Since this function expresses a function relationship between the objective variable and the substitute value, a value of the objective variable can be predicted by inputting the substitute value such as the glucose concentration in the added medium. Therefore, when Formula (1-2) in FIG. 3 is used, the culture tank can be controlled using the substitute value such as the glucose concentration in the added medium.

On the other hand, when the correlation database 300 expressing the correlation between the glucose concentration in the culture tank and the glucose concentration in the added medium, which is a substitute value, is used, Formula (1-3) in FIG. 3, which is the function notation of the objective variable using the explanatory variable such as the glucose concentration in the culture tank as a dependent variable, is obtained. Formula (1-3) in FIG. 3 expresses a biochemical relationship formula related to cell activity. In a case where knowledge is obtained from another experiment, academic paper, or the like in the future, it is also possible to improve the accuracy of the function notation of the objective variable using the substitute value as the dependent variable, by correcting Formula (1-3) in FIG. 3 based on the knowledge and converting the corrected formula into Formula (1-2) in FIG. 3 using the correlation database 300 (Formula (1-1)). Therefore, the accuracy of the control for the culture tank using the substitute value can also be improved.

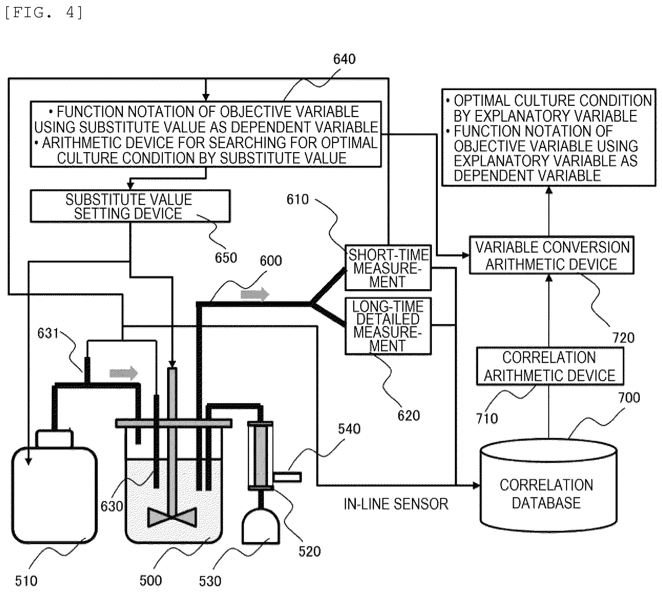

A specific example of the cell culture device according to the embodiment of the invention will be described. FIG. 4 is a diagram showing an example of a configuration of the cell culture device (hereinafter, also referred to as a "culture device").

In the specific example, cells were cultured using the culture device of FIG. 4, and an antibody concentration in a predetermined culture period was measured. As the cells, Chinese hamster ovary cells (CHO cells) were used. These cells are floating cells that secrete the IgG1 antibody. CELLiST BASAL medium was used for culture. A glass cylindrical culture tank having an inner diameter of 110 mm and a culture volume of 2 L was used as the culture device. A heating rubber heater, a magnet driven stirring blade, a temperature measuring electrode, a pH electrode, a DO electrode, and a control device for measuring and adjusting these are connected. A sintered metal sparger having an average pore diameter of 100 µm was incorporated for in-liquid ventilation. A pH of the culture solution was automatically adjusted by increasing or decreasing a concentration of carbon dioxide gas in mixed gas (air, nitrogen, oxygen, carbon dioxide gas) supplied to a gas phase portion of the culture tank. A temperature of the culture solution was adjusted to 37 °C. When the culture solution was acidified, it was adjusted by injecting a sodium hydroxide solution having a concentration of 2%. The dissolved oxygen concentration was adjusted by increasing or decreasing an oxygen partial pressure of gas supplied to a liquid surface gas phase portion and the solution. A drive motor to which a drive shaft, to which a stirring blade was attached, was directly connected was rotated at a rotation speed of 100 rpm. The culture solution in the culture tank was aseptically sampled once a day from the start of culture, and the concentration of the IgG1 antibody was measured. The concentration of the IgG1 antibody was measured using a Cedex Bio Analyzer.

The configuration of the culture device in FIG. 4 is a device configuration of perfusion culture. In the perfusion culture, a medium and cells are placed in a culture tank 500, and the temperature of the culture solution is adjusted to 37 °C to cause the cells to growth. Although nutrients necessary for cell growth are contained in the placed medium, insufficient nutrients due to an increase in the number of cells are supplemented by an added medium prepared in a medium preparation tank 510. At the same time, the medium is extracted by a pump 530 through a cell filtration membrane 520 and discharged from a discharge line 540 to the outside of the culture tank such that a total volume of the medium in the culture tank does not change. At this time, the cells do not permeate the cell filtration membrane 520, but the IgG1 antibody produced by the cells and waste products caused by cell metabolism permeate the cell filtration membrane 520, so that the antibody and waste products can be discharged to the outside of the culture tank.

In order to confirm a state of the cells, a part of the culture solution is periodically extracted from a sampling line 600, and components in the culture solution are subjected to a short-time measurement 610 and a long-time detailed measurement 620. A result of the short-time measurement 610 and data of an in-line sensor 630, which is disposed in any of the culture tank, the medium preparation tank, and a pipe between the medium preparation tank and the culture tank, are sent to the arithmetic device 640.

The arithmetic device 640 searches for, using the mathematical method described above, "a function notation of an objective variable using a substitute value as a dependent variable" and "an optimal culture condition candidate by the substitute value" as a next experiment condition.

At the same time, the arithmetic device 640 determines whether the preset objective variable is maximum or minimum. In this example, it is determined whether the concentration of the IgG1 antibody is maximum. When the objective variable is maximum, a culture condition at the time of determination is determined as "the optimal culture condition by substitute value". When the objective variable is not maximum, the calculated "optimal culture condition candidate" is sent to a substitute value setting device 650, and setting values of the culture tank 500 and the medium preparation tank 510 for the next experiment are changed.

At the same time, a result of the long-time detailed measurement 620, together with the result of the short-time measurement 610 and data of the in-line sensors 630 and 631, is sent to a correlation database 700. Here, since a sensor value of the in-line sensor 630 indicates a value in the culture tank, the sensor value is an explanatory variable. On the other hand, since a sensor value of the in-line sensor 631 indicates a value that indirectly varies a concentration of a medium component in the culture tank, and the sensor value is a substitute value in the invention. The correlation database 700 is accompanied by a correlation arithmetic device 710, which calculates a correlation (Formula (1-1) in FIG. 3) between data stored in the correlation database 700 by the above-described mathematical method, and stores (holds) the correlation as information.

After "the function notation of the objective variable using the substitute value as the dependent variable" and "the optimal culture condition by the substitute value" are obtained by the arithmetic device 640, "the function notation of the objective variable using the explanatory variable as the dependent variable" and "the optimal culture condition by the explanatory variable" are obtained by a variable conversion arithmetic device 720 using Formula (1-3) in FIG. 3, by using the correlation (Formula (1-1) in FIG. 3) between the data stored in the correlation database 700.

The example of the culture device of FIG. 4 is a device configuration of the perfusion culture, but the culture device according to the embodiment of the invention is not limited to the device of the perfusion culture. The culture device according to the embodiment of the invention can be applied to devices of any culture form such as batch culture, fed-batch culture, and continuous culture.

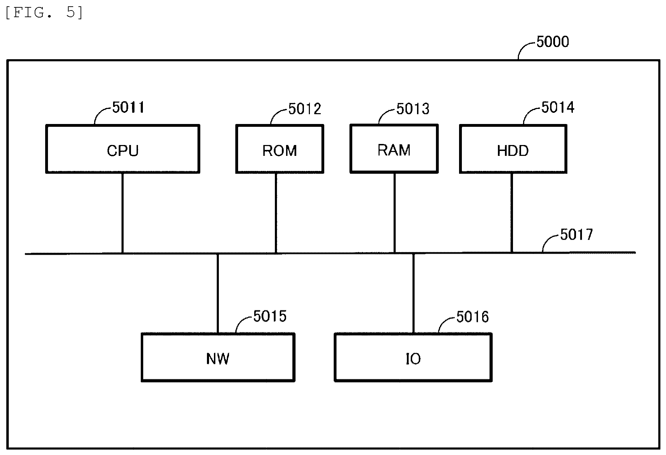

FIG. 5 is a schematic configuration diagram showing an example of a hardware configuration of the arithmetic device. Each of the arithmetic device 640, the correlation arithmetic device 710, and the variable conversion arithmetic device 720 can be implemented by an arithmetic device 5000 shown in FIG. 5. As shown in FIG. 5, the arithmetic device 5000 includes a CPU 5011, an ROM 5012, an RAM 5013, a non-volatile storage device (HDD) 5014 capable of reading and writing data, a network interface 5015, an input and output interface 5016, or the like. These components are communicably connected to one another via a bus 5017. The arithmetic device 5000 may include a plurality of servers or may be a virtual server on a cloud.

The CPU 5011 implements various functions by loading various programs (not shown) stored in the ROM 5012 and/or the HDD 5014 into the RAM 5013 and executing the programs loaded into the RAM 5013. The various programs to be executed by the CPU 5011 is loaded into the RAM 5013 as described above, and data used when the CPU 5011 executes the various programs is temporarily stored in the RAM 5013. The ROM 5012 and/or the HDD5014 is a non-volatile storage medium and stores various programs. The network interface 5015 is an interface for connecting the arithmetic device 5000 to a network. The input and output interface 5016 is an interface for connecting to an operation device such as a keyboard or a mouse and a display (display device). At least a part of processing executed by the CPU 5011 executing the program may be executed by, for example, hardware such as an ASIC or an FPGA.

The arithmetic device 640, the correlation arithmetic device 710, and the variable conversion arithmetic device 720 may be implemented by one arithmetic device 5000 having all of these functions.

The correlation database 700 is stored in a non-volatile storage device (not shown) capable of reading and writing data. The correlation arithmetic device 710 is connected to be accessible to the correlation database 700 stored in the storage device. The substitute value setting device 650 includes a controller (control device) for adjusting (setting) the substitute value or the like. The controller includes a microcomputer having the same configuration as that shown in FIG. 5.

### <Effects>

As described above, the cell culture device according to the embodiment of the invention can quickly determine an optimal culture condition even when the culture condition to be obtained includes a parameter (physical quantity) that takes time for measurement.

In the invention, the search for the optimal culture condition means using a specific measurement value as an objective variable and each of a plurality of culture conditions as an explanatory variable, regarding the objective variable as a function in which the explanatory variables are dependent variables, and using the mathematical method to obtain a combination of the explanatory variables in which the objective variable becomes maximum or minimum, that is, the optimal culture condition based on the explanatory variables.

Among the explanatory variables, an explanatory variable that is a measurement value requiring a long time for measurement is replaced with a substitute value that is a measurement value capable of performing short-time measurement, and the next experiment condition is determined using the substitute value instead of the explanatory variable. By repeating determination of an experiment condition and an experiment in the determined experiment condition, the optimal culture condition based on the substitute values is obtained by obtaining a combination of the substitute values in which the objective variable becomes maximum or minimum. Here, the short time means, for example, a time within 1 hour, and the long time means, for example, a time longer than 1 hour.

After the determination of the optimal culture condition or in parallel with a determination operation, the explanatory variable that is a measurement value requiring a long time for measurement is measured, and a correlation database between the measured explanatory variable and the substitute value is constructed.

By converting the optimal culture condition using the correlation database (correlation), an optimal culture condition based on the explanatory variable can be obtained. In the optimal culture condition search, a function notation of the objective variable using the substitute value as a dependent variable can be simultaneously obtained. By the function notation of the objective variable using the substitute value as the dependent variable and the correlation database between the explanatory variable and the substitute value, a function notation of the objective variable using the explanatory variable as the dependent variable can be obtained. In PTL 1, control is performed using a substitute value, but it is not possible to obtain a function notation expressing a relationship between an objective variable and an explanatory variable that is a source of the substitute value.

### <<Modifications>>

The invention is not limited to the embodiment described above, and various modifications can be adopted within the scope of the invention. For example, the embodiment described above has been described in detail for easy understanding of the invention, and within the scope of the invention, the invention is not necessarily limited to those including all the configurations described above. Further, within the scope of the invention, a part of the configuration according to a certain embodiment can be replaced with a part of the configuration according to another modification, and a configuration according to another modification can be added to a configuration according to a certain embodiment. In addition, within the scope of the invention, another configuration can be added to, deleted from, or replaced with a part of configurations of the device according to one embodiment.

In the above embodiment, the substitute value may be obtained by substituting one parameter with another parameter, may be obtained by substituting one parameter with a plurality of parameters, or may be obtained by substituting a plurality of parameters with one parameter.

### <First Modification>

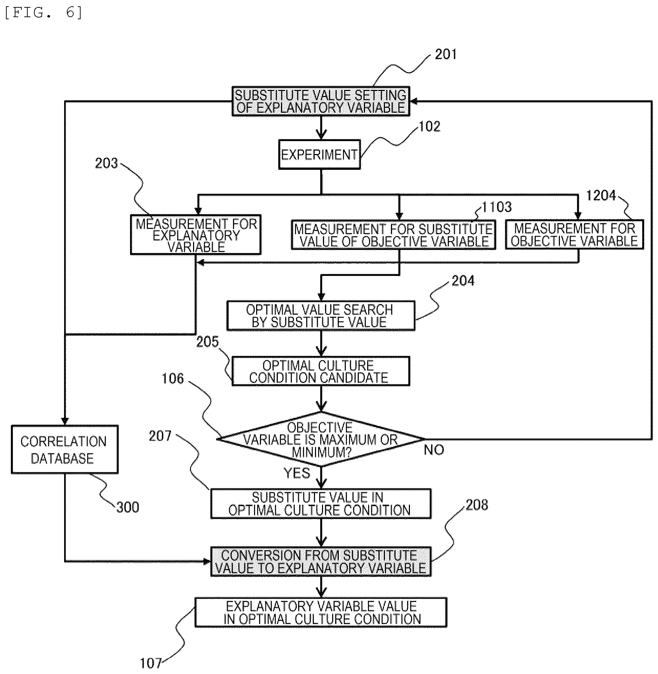

In a first modification of the cell culture device according to the above embodiment, an explanatory variable value determination method in an optimal culture condition may be performed as follows. With reference to FIG. 6, the explanatory variable value determination method in the optimal culture condition performed in the first modification will be described.

In the first modification, first, instead of the explanatory variable that is the culture condition, the substitute value setting 201 of the explanatory variable is performed, and the experiment 102 is performed. The experiment 102 referred to here is to perform culture in the setting of the substitute value setting 201 of the explanatory variable. After the end of the experiment 102, a measurement 1103 for the substitute value of the objective variable and a measurement 1204 for the objective variable are performed.

The measurement 203 for the explanatory variable and the measurement 1204 for the objective variable are performed before, after, or simultaneously with the measurement 1103 for the substitute value of the objective variable. The measurement here includes not only biochemical measurement but also calculation using a result of numerical fluid analysis.

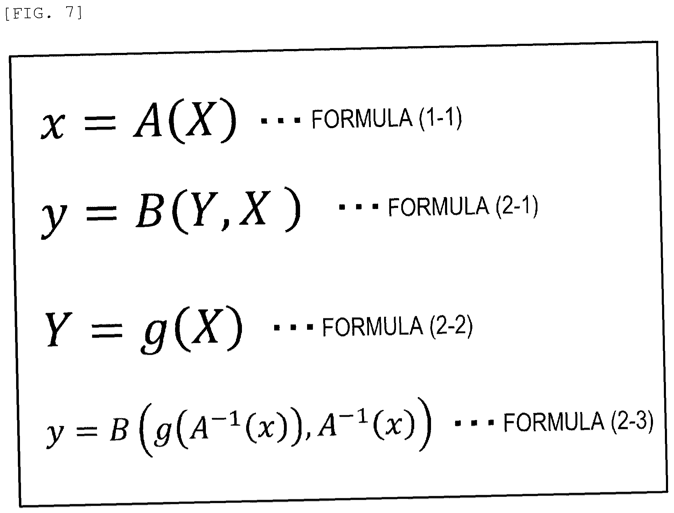

A correlation between "the substitute value setting 201 of the explanatory variable" and "the result of the measurement 203 for the explanatory variable" and a correlation between "the substitute value setting 201 of the explanatory variable and a result of the measurement 1103 for the substitute value of the objective variable" and "a result of the measurement 1204 for the objective variable" are calculated to create the correlation database 300. Methods such as multiple linear regression measurement, nonlinear kernel regression measurement, and support vector regression can be applied to the calculation of the correlation. When a vector in which a plurality of explanatory variables are arranged is x, a vector in which substitute values of the plurality of explanatory variables are arranged is X, a substitute value of the objective variable is Y, and the objective variable is y, these relationships can be expressed by Formula (1-1) in FIG. 7 and Formula (2-1) in FIG. 7.

Based on one or more sets of the substitute value setting 201 of the explanatory variable and the result of the measurement 1103 for the substitute value of the objective variable, the optimal value search 204 by the substitute value (substitute value of the explanatory variable) is performed by using a mathematical method to obtain a function notation of the substitute value of the objective variable using the substitute value of the explanatory variable as a dependent variable. As the mathematical method here, a plurality of methods such as linear regression, kernel regression, and Gaussian process regression can be applied. When the objective variable is y, the vector in which substitute values of the plurality of explanatory variables are arranged is X, and the substitute value of the objective variable is Y, a relationship therebetween can be expressed by Formula (2-2) in FIG. 7.

By using the function notation (Formula (2-2) in FIG. 7) acquired as described above, a substitute value for the next experiment, which is the optimal culture condition candidate 205, is determined using the mathematical method. As the mathematical method here, a Monte Carlo simulation, a genetic algorithm, an annealing method, a Bayesian optimization method, or the like can be applied.

In the determination 106 for the objective variable, a procedure from the substitute value setting 201 of the explanatory variable to the determination of the optimal culture condition candidate 205 is repeated until the substitute value of the objective variable becomes maximum or minimum. The explanatory variable value in which the substitute value of the objective variable becomes maximum or minimum is the substitute value 207 in the optimal culture condition.

The substitute value 207 in the optimal culture condition is converted using Formula (1-1) in FIG. 7 based on the correlation database 300 to obtain the explanatory variable value 107 in the optimal culture condition. At the same time, a function notation of the objective variable expressed by Formula (3-3) in FIG. 7 using the explanatory variable as a dependent variable is obtained by Formulas (1-1) and (2-1) in FIG. 7 and Formula (2-2) in FIG. 7.

According to the first modification, even when the culture condition to be obtained is a parameter (physical quantity) that takes time for measurement, and the objective variable to be measured after the experiment includes a parameter that takes time for measurement, the optimal culture condition can be quickly determined.

### <Second Modification>

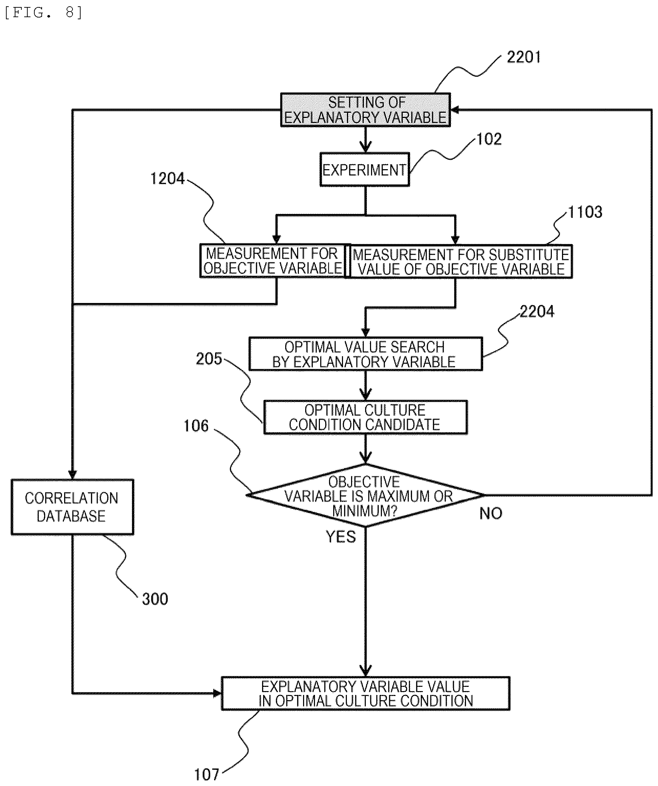

In a second modification of the cell culture device according to the above embodiment, an explanatory variable value determination method in an optimal culture condition may be performed as follows. With reference to FIG. 8, the explanatory variable value determination method in the optimal culture condition performed in the second modification will be described.

In the second modification, a setting 2201 of an explanatory variable that is a culture condition is performed, and the experiment 102 is performed. The experiment 102 referred to here is to perform culture under the culture condition of the explanatory variable. After the end of the experiment 102, the measurement 1103 for the substitute value of the objective variable is performed.

The measurement 1204 for the objective variable is performed before, after, or simultaneously with the measurement 1103 for the substitute value of the objective variable. The measurement here includes not only biochemical measurement but also calculation using a result of numerical fluid analysis.

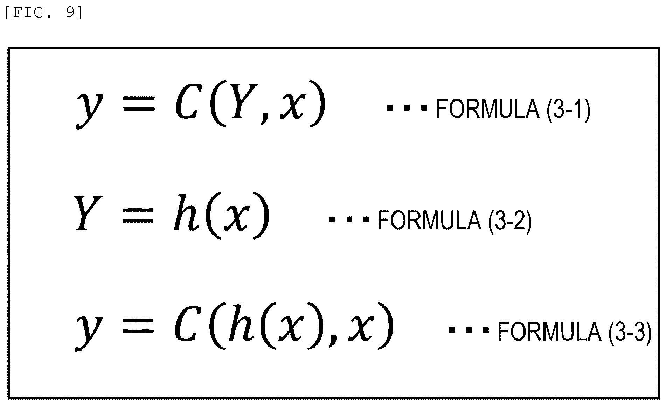

A correlation between the result of the measurement 1103 for the substitute value of the objective variable and the result of the measurement 1204 for the objective variable is calculated to create the correlation database 300. Methods such as multiple linear regression measurement, nonlinear kernel regression measurement, and support vector regression can be applied to the calculation of the correlation. When the objective variable is y, the substitute value of the objective variable is Y, and a vector in which a plurality of explanatory variables are arranged is x, a relationship therebetween can be expressed by Formula (3-1) in FIG. 9.

Based on one or more sets of the explanatory variable and the measurement 1103 for the substitute value of the objective variable, an optimal value search 2204 by the explanatory variable is performed by using a mathematical method to obtain a function notation of the substitute value of the objective variable using the explanatory variable as a dependent variable. As the mathematical method here, a plurality of methods such as linear regression, kernel regression, and Gaussian process regression can be applied. When the substitute value of the objective variable is Y and a vector in which a plurality of explanatory variables are arranged is x, a relationship therebetween can be expressed by Formula (3-2) in FIG. 9.

By using the function notation (Formula (3-2) in FIG. 9) acquired as described above, an explanatory variable (culture condition) for the next experiment, which is the optimal culture condition candidate 205, is determined using the mathematical method. As the mathematical method here, a Monte Carlo simulation, a genetic algorithm, an annealing method, a Bayesian optimization method, or the like can be applied.

In the determination 106 for the substitute value of the objective variable, a procedure from the setting 2201 of the explanatory variable to the determination of the optimal culture condition candidate 205 is repeated until the substitute value of the objective variable becomes maximum or minimum. An explanatory variable value in which the substitute value of the objective variable becomes maximum or minimum is calculated as the optimal culture condition, and the explanatory variable value 107 in the optimal culture condition is obtained. At the same time, a function notation of the objective variable expressed by Formula (3-3) in FIG. 9 using the explanatory variable as a dependent variable is obtained by Formula (3-1) in FIG. 9 and Formula (3-2) in FIG. 9 based on the correlation database 300.

According to the second modification, even when the objective variable to be measured after the experiment includes a parameter that takes time for measurement, the optimal culture condition can be quickly determined.

The invention can also adopt the following configurations.
[1] A cell culture device, which includes a culture tank and a culture solution containing cells inside the culture tank, includes:
   a first measurement device configured to measure a first parameter that is a culture condition and a second parameter related to a result of a culture experiment;
   a second measurement device configured to measure a substitute parameter that is at least one of a first substitute value of the first parameter and a second substitute value of the second parameter; and
   an arithmetic device configured to search for an optimal culture condition, in which
   the arithmetic device
      acquires the first parameter and the second parameter from the first measurement device and acquires the substitute parameter from the second measurement device,
      based on the first parameter, the second parameter, and the substitute parameter,
      obtains a function form expressed using the substitute parameter, the function form using the first parameter as an explanatory variable and the second parameter as an objective variable,
      calculates a parameter correlation between the substitute parameter and at least one of the first parameter and the second parameter substituted by the substitute parameter,
      calculates an optimal explanatory variable in which the objective variable of the function form expressed using the substitute parameter becomes maximum or minimum,
      calculates the calculated optimal explanatory variable as an optimal culture condition when the function form expressed using the substitute parameter is not a function form expressed using the first substitute value, and
      converts the calculated optimal explanatory variable into an optimal first parameter based on the parameter correlation and calculates the optimal first parameter as the optimal culture condition when the function form expressed using the substitute parameter is the function form expressed using at least the first substitute value.
[2] The first measurement device may measure a parameter measurable in a predetermined measurement time or more as the first parameter, and measure a parameter measurable in a measurement time shorter than the predetermined measurement time as the second parameter. The second measurement device may measure a parameter measurable in a measurement time shorter than the predetermined measurement time as the first substitute value.

The first measurement device may measure a parameter measurable in a predetermined first measurement time or more as the first parameter, and measure a parameter measurable in a predetermined second measurement time or more as the second parameter. The second measurement device may measure a parameter measurable in a measurement time shorter than the predetermined first measurement time as the first substitute value, and measure a parameter measurable in a measurement time shorter than the predetermined second measurement time as the second substitute value.

The first measurement device may measure a parameter measurable in a measurement time shorter than a predetermined measurement time as the first parameter, and measure a parameter measurable in the predetermined measurement time or more as the second parameter. The second measurement device may measure a parameter measurable in a measurement time shorter than the predetermined measurement time as the second substitute value.
[3]
   A culture condition determination device includes
   an arithmetic device configured to acquire, from a first measurement device that measures a first parameter that is a culture condition and a second parameter related to a result of a culture experiment and from a second measurement device that measures a substitute parameter that is at least one of a first substitute value of the first parameter and a second substitute value of the second parameter, the first parameter, the second parameter, and the substitute parameter, and search for an optimal culture condition, in which
   the arithmetic device
      obtains, based on the first parameter, the second parameter, and the substitute parameter, a function form expressed using the substitute parameter, the function form using the first parameter as an explanatory variable and the second parameter as an objective variable,
      calculates a parameter correlation between the substitute parameter and at least one of the first parameter and the second parameter substituted by the substitute parameter,
      calculates an optimal explanatory variable in which the objective variable of the function form expressed using the substitute parameter becomes maximum or minimum,
      calculates the calculated optimal explanatory variable as an optimal culture condition when the function form expressed using the substitute parameter is not a function form expressed using the first substitute value, and
      converts the optimal explanatory variable into an optimal first parameter based on the parameter correlation and calculates the optimal first parameter as the optimal culture condition when the function form expressed using the substitute parameter is the function form expressed using at least the first substitute value.
[4]
   A culture condition determination method of cell culture by a culture tank and a culture solution containing cells inside the culture tank, by using
   a first measurement device configured to measure a first parameter that is a culture condition and a second parameter related to a result of a culture experiment,
   a second measurement device configured to measure a substitute parameter that is at least one of a first substitute value of the first parameter and a second substitute value of the second parameter, and
   an arithmetic device configured to search for an optimal culture condition, the culture condition determination method includes:
      by the arithmetic device
      acquiring the first parameter and the second parameter from the first measurement device and acquiring the substitute parameter from the second measurement device;
      based on the first parameter, the second parameter, and the substitute parameter,
      obtaining a function form expressed using the substitute parameter, the function form using the first parameter as an explanatory variable and the second parameter as an objective variable;
      calculating a parameter correlation between the substitute parameter and at least one of the first parameter and the second parameter substituted by the substitute parameter;
      calculating an optimal explanatory variable in which the objective variable of the function form expressed using the substitute parameter becomes maximum or minimum;
      calculating the calculated optimal explanatory variable as an optimal culture condition when the function form expressed using the substitute parameter is not a function form expressed using the first substitute value; and
      converting the calculated optimal explanatory variable into an optimal first parameter based on the parameter correlation and calculating the optimal first parameter as the optimal culture condition when the function form expressed using the substitute parameter is the function form expressed using at least the first substitute value.

### Reference Signs List

- 500:: culture tank
- 510:: medium preparation tank
- 520:: cell filtration membrane
- 530:: pump
- 540:: discharge line
- 600:: sampling line
- 610:: short-time measurement
- 620:: long-time detailed measurement
- 630,: 631: in-line sensor
- 640:: arithmetic device
- 650:: substitute value setting device
- 700:: correlation database
- 710:: correlation arithmetic device
- 720:: variable conversion arithmetic device

## Claims

1. A cell culture device including a culture tank and a culture solution containing cells inside the culture tank, the cell culture device comprising:
a first measurement device configured to measure a first parameter that is a culture condition and a second parameter related to a result of a culture experiment;
a second measurement device configured to measure a substitute parameter that is at least one of a first substitute value of the first parameter and a second substitute value of the second parameter; and
an arithmetic device configured to search for an optimal culture condition, wherein
the arithmetic device
acquires the first parameter and the second parameter from the first measurement device and acquires the substitute parameter from the second measurement device,
based on the first parameter, the second parameter, and the substitute parameter,
obtains a function form expressed using the substitute parameter, the function form using the first parameter as an explanatory variable and the second parameter as an objective variable,
calculates a parameter correlation between the substitute parameter and at least one of the first parameter and the second parameter substituted by the substitute parameter,
calculates an optimal explanatory variable in which the objective variable of the function form expressed using the substitute parameter becomes maximum or minimum,
calculates the calculated optimal explanatory variable as an optimal culture condition when the function form expressed using the substitute parameter is not a function form expressed using the first substitute value, and
converts the calculated optimal explanatory variable into an optimal first parameter based on the parameter correlation and calculates the optimal first parameter as the optimal culture condition when the function form expressed using the substitute parameter is the function form expressed using at least the first substitute value.

2. The cell culture device according to claim 1, wherein
the second measurement device measures the first substitute value as the substitute parameter, and
the arithmetic device
acquires the first parameter and the second parameter from the first measurement device, and acquires the first substitute value as the substitute parameter from the second measurement device,
obtains a first function form, as the function form expressed using the substitute parameter, using the first substitute value as the explanatory variable and the second parameter as the objective variable based on the second parameter and the first substitute value,
calculates a first correlation, as the parameter correlation, between the first substitute value and the first parameter based on the first parameter and the first substitute value,
calculates the optimal explanatory variable in which an objective variable of the first function form becomes maximum or minimum, and
converts the calculated optimal explanatory variable into the optimal first parameter based on the first correlation, and calculates the optimal first parameter as the optimal culture condition.

3. The cell culture device according to claim 1, wherein
the second measurement device measures the first substitute value and the second substitute value as the substitute parameter, and
the arithmetic device
acquires the first parameter and the second parameter from the first measurement device, and acquires the first substitute value and the second substitute value as the substitute parameter from the second measurement device,
obtains a second function form, as the function form expressed using the substitute parameter, using the first substitute value as the explanatory variable and the second substitute value as the objective variable based on the first substitute value and the second substitute value,
calculates a first correlation, as the parameter correlation, between the first substitute value and the first parameter based on the first substitute value and the first parameter, and calculates a second correlation between the second parameter and each of the first substitute value and the second substitute value based on the first substitute value, the second substitute value, and the second parameter,
calculates the optimal explanatory variable in which an objective variable of the second function form becomes maximum or minimum, and
converts the calculated optimal explanatory variable into the optimal first parameter based on the first correlation, and calculates the optimal first parameter as the optimal culture condition.

4. The cell culture device according to claim 1, wherein
the second measurement device measures the second substitute value as the substitute parameter, and
the arithmetic device
acquires the first parameter and the second parameter from the first measurement device, and acquires the second substitute value as the substitute parameter from the second measurement device,
obtains a third function form, as the function form expressed using the substitute parameter, using the first parameter as the explanatory variable and the second substitute value as the objective variable based on the first parameter and the second substitute value, and
calculates the optimal explanatory variable in which an objective variable of the third function form becomes maximum or minimum, and calculates the calculated optimal explanatory variable as the optimal culture condition.

5. The cell culture device according to claim 1, wherein
the arithmetic device converts the function form when the objective variable is maximum or minimum into the function form using the first parameter as the explanatory variable and the second parameter as the objective variable based on the parameter correlation.

6. The cell culture device according to claim 2, wherein
the first measurement device measures a parameter measurable in a predetermined measurement time or more as the first parameter, and measures a parameter measurable in a measurement time shorter than the predetermined measurement time as the second parameter, and
the second measurement device measures a parameter measurable in a measurement time shorter than the predetermined measurement time as the first substitute value.

7. The cell culture device according to claim 6, wherein
the first measurement device is disposed inside the culture tank, and
the second measurement device is disposed outside the culture tank.

8. The cell culture device according to claim 3, wherein
the first measurement device measures a parameter measurable in a predetermined first measurement time or more as the first parameter, and measures a parameter measurable in a predetermined second measurement time or more as the second parameter, and
the second measurement device measures a parameter measurable in a measurement time shorter than the predetermined first measurement time as the first substitute value, and measures a parameter measurable in a measurement time shorter than the predetermined second measurement time as the second substitute value.

9. The cell culture device according to claim 4, wherein
the first measurement device measures a parameter measurable in a measurement time shorter than a predetermined measurement time as the first parameter, and measures a parameter measurable in the predetermined measurement time or more as the second parameter, and
the second measurement device measures a parameter measurable in a measurement time shorter than the predetermined measurement time as the second substitute value.

10. The cell culture device according to claim 2, wherein
the second parameter is a parameter measured based on the result of the culture experiment, in which the first substitute value is set as an experiment condition, by performing the culture experiment in which the first substitute value is set as the experiment condition, and
the arithmetic device
searches for the optimal explanatory variable based on the first substitute value and the second parameter acquired every time the culture experiment is performed by repeatedly performing the culture experiment until the objective variable of the first function form becomes maximum or minimum when calculating the optimal explanatory variable in which the objective variable of the first function form becomes maximum or minimum, and
calculates the first substitute value suitable for a next experiment based on the acquired first substitute value and the first function form.

11. A culture condition determination device comprising:
an arithmetic device configured to acquire, from a first measurement device that measures a first parameter that is a culture condition and a second parameter related to a result of a culture experiment and from a second measurement device that measures a substitute parameter that is at least one of a first substitute value of the first parameter and a second substitute value of the second parameter, the first parameter, the second parameter, and the substitute parameter, and search for an optimal culture condition, wherein
the arithmetic device
obtains, based on the first parameter, the second parameter, and the substitute parameter, a function form expressed using the substitute parameter, the function form using the first parameter as an explanatory variable and the second parameter as an objective variable,
calculates a parameter correlation between the substitute parameter and at least one of the first parameter and the second parameter substituted by the substitute parameter,
calculates an optimal explanatory variable in which the objective variable of the function form expressed using the substitute parameter becomes maximum or minimum,
calculates the calculated optimal explanatory variable as an optimal culture condition when the function form expressed using the substitute parameter is not a function form expressed using the first substitute value, and
converts the optimal explanatory variable into an optimal first parameter based on the parameter correlation and calculates the optimal first parameter as the optimal culture condition when the function form expressed using the substitute parameter is the function form expressed using at least the first substitute value.

12. A culture condition determination method of cell culture by a culture tank and a culture solution containing cells inside the culture tank, by using
a first measurement device configured to measure a first parameter that is a culture condition and a second parameter related to a result of a culture experiment,
a second measurement device configured to measure a substitute parameter that is at least one of a first substitute value of the first parameter and a second substitute value of the second parameter, and
an arithmetic device configured to search for an optimal culture condition, the culture condition determination method comprising:
by the arithmetic device
acquiring the first parameter and the second parameter from the first measurement device and acquiring the substitute parameter from the second measurement device;
based on the first parameter, the second parameter, and the substitute parameter,
obtaining a function form expressed using the substitute parameter, the function form using the first parameter as an explanatory variable and the second parameter as an objective variable;
calculating a parameter correlation between the substitute parameter and at least one of the first parameter and the second parameter substituted by the substitute parameter;
calculating an optimal explanatory variable in which the objective variable of the function form expressed using the substitute parameter becomes maximum or minimum;
calculating the calculated optimal explanatory variable as an optimal culture condition when the function form expressed using the substitute parameter is not a function form expressed using the first substitute value; and
converting the calculated optimal explanatory variable into an optimal first parameter based on the parameter correlation and calculating the optimal first parameter as the optimal culture condition when the function form expressed using the substitute parameter is the function form expressed using at least the first substitute value.
